# EUROPÄISCHE PATENTSCHRIFT

(11) **EP 2 467 414 B1**
(45) Veröffentlichungstag und Bekanntmachung des Hinweises auf die Patenterteilung: **16.04.2014**
(21) Anmeldenummer: 10742168.7
(22) Anmeldetag: 16.08.2010
(51) Int. Cl.: C08G 63/82, C08G 63/87, C08G 63/06, C08G 63/12, C08G 18/42

(54) **VERFAHREN ZUR HERSTELLUNG VON POLYESTERALKOHOLEN**
PROCESS FOR MAKING POLYESTER POLYOL
PROCÉDÉ DE PRÉPARATION DE POLYESTERPOLYOL

(30) Priorität: 20.08.2009 EP 09168315
(43) Veröffentlichungstag der Anmeldung: 27.06.2012
(73) Patentinhaber: BASF SE, 67056 Ludwigshafen (DE)
(72) Erfinder: GEHRINGER, Lionel, F-67470 Schaffhouse-pres-Seltz (FR); WLOKA, Veronika, 68163 Mannheim (DE); MÜLLER, Ulrich, 67435 Neustadt (DE); BOUDOU, Marine, 68161 Mannheim (DE); MAHN, Ulrike, 68259 Mannheim (DE); GÜTLICH-HAUK, Elke, 67245 Lambsheim (DE)
(86) Internationale Anmeldenummer: PCT/EP2010/061895
(87) Internationale Veröffentlichungsnummer: WO 2011/020815

(56) Entgegenhaltungen:
- EP-A1- 2 154 221
- WO-A1-2010/035579
- WO-A1-2010/083957
- DE-A1-102008 004 343
- US-A- 5 733 969
- US-A- 5 952 455
- US-A1- 2006 036 054

## Beschreibung

Die Erfindung betrifft ein Verfahren zur Herstellung von Polyesteralkoholen.

Die Herstellung von Polyesteralkoholen und der Einsatz derartiger Produkte in der Polyurethanchemie sind seit langem bekannt und vielfach beschrieben. Zumeist werden diese Produkte durch Polykondensationsreaktionen von mehrwertigen Carbonsäuren und/oder Carbonsäurederivaten mit mehrwertigen Alkoholen bzw. Polyolen hergestellt. Beispielhaft genannt sei das Kunststoffhandbuch, Band VII, Polyurethane, Carl- Hanser-Verlag, München 1. Auflage 1966, herausgegeben von Dr. R Vieweg und Dr. A. Höchtlen, sowie 2. Auflage 1983 und die 3. neubearbeitete Auflage 1993, herausgegeben von Dr. G. Oertel. Es ist auch bekannt, Polyesteralkohole durch Polykondensationsreaktionen von ω-Hydroxycarbonsäure oder durch Ringöffnungspolymerisation von cyclischen Estern, so genannten Lactonen, herzustellen.
Es ist aber auch möglich, Polyesterabfälle und insbesondere Polyethylenterephthalat (PET)- bzw. Polybutylenterephthalat (PBT)-Abfälle zu verarbeiten. Hierfür sind eine ganze Reihe von Verfahren bekannt und beschrieben. Grundlage einiger Verfahren ist die Umwandlung des Polyesters in einen Diester der Terephthalsäure, z.B. in Dimethylterephthalat. In DE-A 1003714 bzw. US-A 5,051,528 werden derartige Umesterungen unter Einsatz von Methanol und Umesterungskatalysatoren beschrieben.

Die Anwendung dieser Polyesteralkohole insbesondere zur Herstellung von Polyurethanen, im folgenden auch als PUR bezeichnet, insbesondere von PUR-Weichschaum, PUR-Hartschaum, sowie anderen zelullären oder nicht zellulären PUR-Materialien bedingt eine konkrete Auswahl der Einsatzprodukte und der durchzuführenden Polykondensationstechnologie. Zur Herstellung von Polyurethan ist insbesondere wichtig, dass die eingesetzten Polyesteralkohole eine niedrige Säurezahl besitzen (siehe Ullmann's Encyclopedia, Electronic Release, Wiley-VCH-Verlag GmbH, Weinheim, 2000 unter dem Stichwort "polyesters", Absatz 2.3 "Quality Specifications and Testing"). Die Säurezahl sollte möglichst klein sein, da die endständigen Säuregruppen langsamer mit Diisocyanaten reagieren als endständige Hydroxylgruppen. Polyesteralkohole mit hohen Säurezahlen führen daher zu einem geringeren Molekulargewichtsaufbau während der Umsetzung von Polyesteralkoholen mit Isocyanaten zum Polyurethan.

Ein weiteres Problem beim Einsatz von Polyesteralkoholen mit hohen Säurezahlen zur Polyurethan-Reaktion ist, dass bei der Reaktion der zahlreichen endständigen Säuregruppen mit Isocyanaten eine Amidbindung unter Freisetzung von Kohlendioxid bildet. Das gasförmige Kohlendioxid kann dann zu einer unerwünschten Blasenbildung führen. Weiterhin verschlechtern freie Carboxylgruppen die Katalyse bei der Polyurethan-Reaktion und auch die Stabilität der hergestellten Polyurethane gegenüber Hydrolyse.

Bekannte Polykondensationstechnologie zur Herstellung von Polyesteralkohole ist der Einsatz mehrfunktioneller aromatischer und/oder aliphatischer Carbonsäuren bzw. deren Anhydride und di-, tri- und /oder höherfunktionellen Alkoholen, insbesondere von Glykolen, die bei Temperaturen von insbesondere 150-280°C unter Normaldruck und oder leichtem Vakuum im Beisein von Katalysatoren miteinander unter Entzug des Reaktionswassers zur Umsetzung gebracht werden. Die übliche Technologie ist z. B. in DE-A-2904184 beschrieben und besteht in der Zugabe der Reaktionskomponenten zu Synthesebeginn mit einem geeigneten Katalysator bei gleichzeitiger Temperaturerhöhung und Druckabsenkung. Die Temperaturen und das Vakuum werden dann im Laufe der Synthese weiter verändert. Die Polykondensationsreaktionen können sowohl in Gegenwart als auch in Abwesenheit eines Lösungsmittels durchgeführt werden.

Einen Nachteil stellt die Tatsache dar, dass bei der Polykondensationsreaktion bei hohen Temperaturen häufig Nebenprodukte gebildet werden. Weiterhin müssen die Hochtemperatur-Polykondensationen unter Ausschluss von Wasser stattfinden, um die Rückreaktion zu vermeiden. Dies wird in der Regel dadurch erreicht, dass die Kondensation im Vakuum, unter Inertgas-Atmosphäre oder in Anwesenheit eines Schleppgases zur vollständigen Entfernung des Wassers durchgeführt wird.

Ein weiterer Nachteil dieser Polykondensationen bei hohen Temperaturen ist, dass sie verhältnismäßig langsam ablaufen. Um die Polykondensationsreaktion bei hohen Temperaturen zu beschleunigen, werden daher häufig Veresterungskatalysatoren eingesetzt. Als klassische Veresterungskatalysatoren kommen beispielsweise Eisen-, Cadmium-, Kobalt-, Blei-, Zink-, Zirkonium-, Hafnium-, Aluminium-, Antimon-, Magnesium-, Titan- und Zinnkatalysatoren in Form von Metallen, Metalloxiden oder Metallsalzen in Betracht, oder Säuren, wie z.B. Schwefelsäure, p-Toluolsulfonsäure oder Basen, wie z.B. Kaliumhydroxid oder Natrium-Methanolat.

Diese Veresterungskatalysatoren sind homogen und verbleiben nach Beendigung der Reaktion in der Regel im Polyesteralkohol. Nachteilig dabei ist, dass die im Polyesteralkohol verbleibenden Veresterungskatalysatoren gegebenenfalls die Stabilität der hergestellten Polyesteralkohole gegenüber Hydrolyse sowie die spätere Umsetzung dieser Polyesteralkohole zum Polyurethan beeinträchtigen können.

Ferner kann die Anwesenheit der homogenen Katalysatoren im Polyesteralkohol zu Verfärbungen führen.

Um diesen Nachteil zu beheben, wird in WO 2006/100231 ein Verfahren zur Herstellung von Polyesteralkoholen beschrieben, bei dem als Katalysatoren Enzyme eingesetzt werden. Die Herstellung beziehungsweise Umesterung von Polyesteralkoholen mittels Enzymen kann batchweise oder kontinuierlich durchgeführt werden. Bei der kontinuierlichen Verfahrensweise liegt der Katalysator vorzugsweise immobilisiert vor, wobei die Umsetzung vorzugsweise in einem Strömungsreaktor durchgeführt wird.

Den Hochtemperatur-Polykondensationen und den enzymatisch katalysierten Polykondensationen zur Herstellung von Polyesteralkoholen ist jedoch der Nachteil gemeinsam, dass die Herstellung von Polyesteralkoholen durch Kondensationsreaktionen in Anlagen durchgeführt wird, für die eine aufwendige Peripherie erforderlich ist. Für die klassische Hochtemperatur-Polykondensation, wie auch für die enzymatische Polykondensation sind Vorrichtungen am Reaktor für die Dosierung von Flüssigkeiten und/oder Feststoffen erforderlich. Wasser muss unter Vakuum, Einleiten eines Inertgases oder durch eine Schleppmitteldestillation aus dem Reaktionsgemisch entfernt werden. Zudem muss das Wasser von den Diolen destillativ abgetrennt werden, da diese als Reaktionspartner der Säurekomponente im Reaktionsgemisch verbleiben müssen. Die Abtrennung von Wasser und Diolen erfolgt in der Regel mit einer Destillationskolonne. Vorrichtungen zur Erzeugung von Vakuum, z.B. Pumpen, zur Trennung von Diolen und Wasser, z.B. Destillationskolonnen, oder zur Einleitung eines Inertgasstromes erfordern hohe Investitionen. Insbesondere bei der Hochtemperaturkondensation sind außerdem noch Vorrichtungen zur Erzeugung von Reaktorinnentemperaturen von 160-270°C erforderlich.

Nachteile der enzymatischen Katalysatoren sind deren hoher Preis und die Beeinträchtigung des Geruchs und der Farbe der resultierenden Polyesteralkohole. Weiterhin kann es zu einer Ablösung der Enzyme von deren Träger kommen.

In DE 10 2008 004 343 wird ein Verfahren zur Herstellung von Polyesteralkoholen beschrieben, bei dem als heterogene Katalysatoren Multimetallcyanidkatalysatoren, auch als DMC-Katalysatoren bezeichnet, eingesetzt werden. Derartige Katalysatoren sind bekannt und werden häufig als Katalysatoren für die Herstellung von Polyetheralkoholen durch Anlagerung von Alkylenoxiden an Verbindungen mit reaktiven Wasserstoffatomen eingesetzt. Bei der Herstellung von Polyesteralkoholen hat sich gezeigt, dass DMC-Katalysatoren wenig geeignet sind, da ihre katalytische Aktivität unzureichend ist und keine homogenen Produkte erhalten wurden.

EP 1679322 beschreibt ein Verfahren zur Herstellung von Polyestern durch Umsetzung von mehrfunktionellen Alkoholen mit mehrfunktionellen Carbonsäuren. Als Katalysatoren werden Metallsilikate eingesetzt. Bei den in diesem Dokument beschriebenen Produkten handelt es sich nicht um Polyesteralkohole, sondern um thermoplastische Produkte. Durch den Einsatz der genannten Katalysatoren sollte die Herstellung von Produkten mit sehr hohem Molekulargewicht erleichtert werden. Weiterhin sollte das Verhalten bei der thermoplastischen Verarbeitung der Produkte und die mechanischen

Eigenschaften der Endprodukte verbessert werden, insbesondere sollte die Tendenz zum thermischen Abbau der Produkte während der Verarbeitung verringert werden

Es war die Aufgabe der vorliegenden Erfindung, ein Verfahren zur katalytischen Herstellung von Polyesteralkoholen unter Einsatz von heterogenen Katalysatoren zu entwickeln, das einfach und kostengünstig ist. Das Verfahren sollte zu farblosen und katatysatorfreien Produkten führen, die ohne aufwendige Aufarbeitung zur Herstellung von Polyurethanen eingesetzt werden können. Weiterhin sollte es möglich sein, den Katalysator auch als Festbett einzusetzen.

Die Aufgabe konnte überraschenderweise dadurch gelöst werden, dass als Katalysator für die Herstellung der Polyesteralkohole ein Zeolith eingesetzt wird.

Gegenstand der Erfindung ist demzufolge ein Verfahren zur Herstellung von Polyesteralkoholen durch katalytische Umsetzung von mindestens einer mehrfunktionellen Carbonsäure mit mindestens einem mehrfunktionellen Alkohol und/oder durch katalytische Ring-Öffnungs-Polymerisation von cyclischen Estern, vorzugsweise Lactonen, insbesondere ε-Caprolacton, in Gegenwart von Katalysatoren, dadurch gekennzeichnet, dass als Katalysator ein Zeolith eingesetzt wird, wobei das Zeolith ein Titanzeolith ist.

In einer Ausführungsform des erfindungsgemäßen Verfahrens wird die gesamte Umsetzung der Monomere zum Polyesteralkohol unter Verwendung von Zeolithen durchgeführt.

Es ist jedoch auch möglich, nur einen Teil der Umsetzung unter Verwendung von Zeolithen durchzuführen. Der übrige Teil der Umsetzung kann unkatalysiert oder unter Verwendung anderer Veresterungskatalysatoren durchgeführt werden.

Zeolithe sind bekanntermaßen kristalline Alumosilikate mit geordneten Kanal- und Käfigstrukturen, deren Porenöffnungen im Bereich von Mikroporen liegen. Gemäß IUPAC versteht man unter Mikroporen solche Poren, deren Durchmesser kleiner als 2 nm sind. Das Netzwerk solcher Zeolithe ist aufgebaut aus Si0₄- und AlO₄-Tetraedern, die über gemeinsame Sauerstoffbrücken verbunden sind. Analog gibt es aus PO4- und AlO4-Tetraedern aufgebaute Alumophosphate sog. AlPOs, MeAPOs, MeAPSOs mit zeolithanaloger Struktur, ebenso wie es Metall-imidazolate sog. ZIFs mit zeolithanaloger Struktur gibt. Diese zeolithanalogen Materialien sind vollumfänglich in der vorliegenden Erfindung inbegriffen. Eine Übersicht der bekannten Strukturen findet sich beispielsweise bei Ch. Baerlocher, W.M. Meier, D.H. Olson, "Atlas of Zeolite Framework Types", 5th ed. Elsevier, 2001.

Es sind auch Zeolithe bekannt, die kein Aluminium enthalten und bei denen im Silicatgitter anstelle des Si(IV) teilweise Titan als Ti(IV) steht. Diese Titanzeolithe, insbesondere solche mit einer Kristallstruktur vom MFI-Typ, sowie Möglichkeiten zu ihrer Herstellung sind beschrieben, beispielsweise in der EP-A 311 983 oder der EP-A 405 978. Außer Silizium und Titan können solche Materialien auch zusätzliche Elemente wie Aluminium, Zirkonium, Germanium, Zinn, Eisen, Kobalt, Nickel, Gallium, Bor oder geringe Mengen an Fluor enthalten.

Es werden als Zeolithe solche eingesetzt, die Titan enthalten, im Folgenden als Titanzeolithe bezeichnet. Der Begriff Titanzeolith bezeichnet somit ein Material, welches geringe Mengen Titan neben Siliziumoxid und aufgebaut in einer Zeolithstruktur enthält.

Bevorzugte Titanzeolithe sind solche mit Pentasiteinheiten in der Struktur, z.B. MFI, MEL, BEA, MOR, MWW-Struktur, insbesondere die Typen mit röntgenographischer Zuordnung zur MFI, MOR, BEA, MWW, RRO, LEV, FER-Struktur, insbesondere zur MFI-, MEL- oder MFI/MEL-Mischstruktur. Zeolithe dieses Typs sind beispielsweise in "Atlas of Zeolite Framework Types", Ch. Baerlocher, W.M. Meier, D.H. Olson, 5th ed. Elsevier, 2001 beschrieben.

Der Titanzeolith kann als Festbett oder als Pulver eingesetzt werden.

Üblicherweise stellt man die genannten Titanzeolithe dadurch her, dass man eine wässrige Mischung aus einer SiO₂-Quelle, einer Titan-Quelle wie Titandioxid oder Titanalkoholat und einem stickstoffhaltigen organischen Templat zur Strukturausbildung z.B. Tetrapropylammoniumhydroxid, gegebenenfalls noch unter Hinzufügen von Alkalimetallverbindungen, in einem Druckbehälter unter erhöhter Temperatur im Zeitraum weniger Stunden oder einiger Tage umsetzt, wobei das kristalline Produkt entsteht. Dieses wird abgetrennt, z.B. abfiltriert, gewaschen, getrocknet und zur Entfernung der organischen Stickstoffbase bei erhöhter Temperatur calciniert. In den erfindungsgemäßen Katalysatoren für die Herstellung von Polyesteralkoholen liegt das molare Verhältnis von Titan zur Summe aus Silizium plus Titan in der Regel im Bereich von 0,01:1 bis 0,1:1. In dem so erhaltenen Pulver liegt das Titan zumindest teilweise innerhalb des Zeolithgerüsts in wechselnden Anteilen mit vier-, fünf- oder sechsfacher Koordination vor. Titanzeolithe mit MFI-Struktur sind dafür bekannt, dass sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Die so hergestellten Titanzeolithe können in Form von Pulvern, Sprühagglomeraten oder in Form von Formkörpern wie Strängen, Split, Ringen, Hohlzylindern, Kugeln oder Tabletten eingesetzt werden. Als Formgebungsprozess können im Prinzip alle Methoden zur entsprechenden Formung verwendet werden, wie sie bei Katalysatoren allgemein üblich sind. Bevorzugt werden Prozesse, bei denen die Formgebung durch Extrusion in üblichen Extrudern, beispielsweise zu Strängen mit einem Durchmesser von üblicherweise 1 bis 10 mm, insbesondere 2 bis 5 mm, erfolgt. Werden Bindemittel und/oder Hilfsmittel benötigt, ist der Extrusion zweckmäßigerweise ein Mischungsoder Knetprozess vorgeschaltet. Gegebenenfalls erfolgt nach der Extrusion noch ein Kalzinierungsschritt. Die erhaltenen Stränge werden gegebenenfalls zerkleinert, vorzugsweise zu Granulat oder Split mit einem Partikeldurchmesser von 0,1 bis 5 mm, insbesondere 0,5 bis 2 mm.

Als Bindemittel eignen sich im Prinzip alle für derartige Zwecke eingesetzte Verbindungen; bevorzugt werden Verbindungen, insbesondere Oxide, des Siliziums, Aluminiums, Bors, Phosphors, Zirkons und/oder Titans oder auch Tone, z.B. Montmorillonite, Kaoline oder Bentonite oder auch andere Zeolithe. Von besonderem Interesse als Bindemittel ist Siliziumdioxid, wobei das SiO₂ als Kieselsol oder in Form von Tetraalkoxysilanen in den Formgebungsschritt eingebracht werden kann. Als Hilfsmittel für die verfestigenden Formgebungsprozesse sind beispielsweise Verstrangungshilfsmittel für die Extrusion zu nennen, ein übliches Verstrangungshilfsmittel ist Methylcellulose. Derartige Mittel werden in der Regel in einem nachfolgenden Kalzinierungsschritt vollständig verbrannt.

Die zu Formkörpern verarbeiteten Katalysatoren enthalten bis zu 50 Gew.-% Bindemittel, bezogen auf die Gesamtmasse des Katalysators, bevorzugte Bindemittelgehalte sind 0,1 bis 30 Gew.-%, besonders bevorzugt 2 bis 25 Gew.-%.

Die Katalysatoren werden in der Regel vor dem Einsatz durch erhöhte Temperatur, bevorzugt bei 100 bis 800 °C, besonders bevorzugt bei 200 bis 600 °C und ansonsten dem Fachmann bekannten Bedingungen aktiviert. In vielen Fällen ist die Regeneration der Katalysatoren durch Reaktion mit Luft, Magerluft, das ist ein Gemisch aus Stickstoff und Sauerstoff mit einem Anteil an Sauerstoff, der kleiner als der der Luft ist, oder durch Extraktion oder Spülen mit organischen Lösemitteln oder Wasser möglich.

Die Katalysatoren werden bei der Verwendung als Pulver vorzugsweise in einer Menge von 0,001 bis 1 Gew.-%, bezogen auf das Gewicht des Polyesteralkohols eingesetzt. Bevorzugt liegt die Menge des Katalysators zwischen 0,15 bis 0,25 Gew.-%, bezogen auf das Gewicht des Polyesteralkohols.

Nach der Umsetzung wird der Katalysator aus dem Produkt entfernt. Dies kann vorzugsweise mittels Filtration erfolgen.

Nach der Reinigung enthält der Polyesteralkohol zumeist weniger als 1 ppm Titan und weniger als 100 ppm Silizium, vorzugsweise weniger als 0,5 ppm Titan und 50 ppm Silizium, besonders bevorzugt weniger als 0.2 ppm Titan und 20 ppm Silizium, und insbesondere von weniger als 0.1 ppm Titan und 10 ppm Silizium.

Der abgetrennte Katalysator kann wieder für das Verfahren eingesetzt werden. Dabei kann vor der Wiederverwendung eine Reinigung vom anhaftenden Polyesteralkohol erfolgen.

Prinzipiell könnte der Katalysator auch im Polyesteralkohol belassen werden Dies ist jedoch nicht bevorzugt, da dies bei der Weiterverarbeitung zu Polyurethanen, insbesondere zu thermoplastischen Polyurethanen (TPU) zu Problemen führen kann.

Die Umsetzung der Ausgangsstoffe zum Polyesteralkohol in Anwesenheit der beschriebenen Katalysatoren wird unter den hierfür üblichen Bedingungen durchgeführt.

Vorzugsweise erfolgt die Herstellung der Polyesteralkohole, wie beschreiben, durch Umsetzung von mehrfunktionellen Carbonsäuren mit mehrfunktionellen Alkoholen.

Die Herstellung der Polyesteralkohole kann ein- oder zweistufig durchgeführt werden. Bei der einstufigen Fahrweise wird während der gesamten Umsetzung die Veresterung der Carbonsäuren und Alkohole durchgeführt und danach der fertige Polyesteralkohol entnommen. Bei der zweistufigen Umsetzung wird in einer ersten Stufe a) ein Polyesteralkohol hergestellt und dieser in einer zweiten Stufe b) mit weiteren Carbonsäuren und Alkoholen oder mit einem Polyesteralkohol umgesetzt.

In einer bevorzugten Ausführungsform wird die Umsetzung zweistufig durchgeführt und umfasst folgende Verfahrensschritte:
a) Herstellung mindestens eines Basis-Polyesteralkohols durch die Umsetzung von jeweils mindestens einer Dicarbonsäure mit jeweils mindestens einer Polyhydroxylverbindung
b) Umsetzung des Produkts aus Schritt a) oder einer Mischung aus dem Produkt aus Schritt a), gegebenenfalls im Gemisch mit weiteren Polyhydroxylverbindungen, mit einem Titanzeolith.

Bevorzugt wird dabei Schritt a) in Anwesenheit eines Veresterungskatalysators durchgeführt. Der Veresterungskatalysator wird dabei bevorzugt ausgewählt aus der Gruppe enthaltend Toluolsulfonsäuren und metallorganische Verbindungen. Bevorzugt sind dabei metallorganische Verbindungen auf Basis von Titan oder Zinn.
Dabei sind die metallorganischen Verbindungen Titantetrabutylat oder Zinn-(II)-octoat, Dibutylzinnlaurat und/oder Zinnchlorid.

In einer bevorzugten Ausführungsform wird beim zweistufigen Verfahren der Schritt b) kontinuierlich durchgeführt.

Dabei kann die gesamte Umsetzung oder ein Teil der Umsetzung mit den Zeolith-Katalysatoren durchgeführt werden. Bei der zweistufigen Umsetzung kann eine Stufe mit einem Zeolith-Katalysator und die andere mit einem anderen Katalysator durchgeführt werden. Falls so verfahren wird, ist es bevorzugt, die zweite Stufe unter Verwendung eines Zeolith-Katalysators durchzuführen.

Die nach dem erfindungsgemäßen Verfahren hergestellten Polyesteralkohole weisen, je nach dem gewünschten Verwendungszweck, eine Hydroxylzahl im Bereich zwischen 20 und 400 mgKOH/g auf. Dabei liegt die Hydroxylzahl von Polyesteralkoholen, die für die Herstellung von Polyurethan-Weichschaumstoffen sowie zelligen oder thermoplastischen Polyurethan-Elastomeren eingesetzt werden, vorzugsweise im Bereich zwischen 20 und 250 mgKOH/g. Polyesteralkohole für den Einsatz in Polyurethan-Hartschaumstoffen weisen vorzugsweise eine Hydroxylzahl von über 100 mgKOH/g, insbesondere zwischen 100 und 400 mgKOH/g auf.

Als mehrfunktionelle Carbonsäuren werden zumeist Dicarbonsäuren, wie aliphatische Dicarbonsäuren, vorzugsweise Bernsteinsäure, Glutarsäure, Adipinsäure, Korksäure, Azelainsäure, Sebacinsäure, Decandicarbonsäure, Maleinsäure, Fumarsäure, oder andere aliphatische Dicarbonsäuren, oder aromatische Dicarbonsäuren, wie Phthalsäure, Isophthalsäure oder Terephthalsäure eingesetzt. Die Dicarbonsäuren können dabei sowohl einzeln als auch im Gemisch untereinander verwendet werden. An Stelle von oder im Gemisch mit den Dicarbonsäuren können auch die entsprechenden Dicarbonsäurederivate, wie z.B. Dicarbonsäureester von Alkoholen mit 1 bis 4 Kohlenstoffatomen oder deren Anhydride, beispielsweise Phthalsäureanhydrid, eingesetzt werden.

Als Polyhydroxylverbindung eignen sich sämtliche mindestens zweiwertigen Alkohole, jedoch vorzugsweise Diol-Komponenten wie z.B. Ethylenglykol, Diethylenglykol, 1,3-Propandiol, 1,2-Propandiol, Dipropylenglykol, 1,4-Butandiol, 1,5-Pentandiol, 1,6-Hexandiol, 1,10-Decandiol, Neopentylglykol, 2-Methyl-1,3-Propandiol, 3-Methyl-1,5-Pentandiol. Zur Erhöhung der Funktionalität der Polyesteralkohole können auch dreioder höherfunktionelle Alkohole eingesetzt werden. Beispiele hierfür sind Glycerin, Trimethylolpropan und Pentaerythrit. Möglich ist auch der Einsatz oligomerer oder polymerer Produkte mit mindestens zwei Hydroxylgruppen. Beispiele hierfür sind Polytetrahydrofuran, Polylactone, Polyglycerol, Polyetherole, Polyesterol oder α,ω-Dihyxdroxypolybutadien..

Zur Herstellung der Polyesteralkohole werden die organischen Polycarbonsäuren und/oder -derivate und mehrwertigen Alkohole vorzugsweise im Molverhältnis von 1:1 bis 2,1, vorzugsweise 1:1,05 bis 1,9, polykondensiert.

Die einstufige Herstellung der Polyesteralkohole beziehungsweise die Reaktionsstufe a) der zweistufigen Herstellung der Polyesteralkohole wird, wie beschrieben, durch Umsetzung der mehrfunktionellen Carbonsäuren mit den mehrfunktionellen Alkoholen unter Entfernung des Wassers durchgeführt. Vorzugsweise wird zur Durchführung des Verfahrensschrittes a) ein Rührkesselreaktor mit Rührwerk und Destillationskolonne verwendet. Diese Apparatur stellt in der Regel ein geschlossenes System dar und kann im Allgemeinen mit Hilfe einer Vakuumpumpe evakuiert werden. Die Edukte werden unter Rühren und vorzugsweise unter Luftausschluss (z.B. in Stickstoff-Atmosphäre oder bei reduziertem Druck) erhitzt. Das bei der Polykondensation entstehende Wasser wird vorzugsweise bei niedrigem Druck bzw. einem kontinuierlich kleiner werdenden Druck abdestilliert (siehe Batchwise Vacuum-Melt-Verfahren, Houben-Weyl 14/2, 2).

Die Reaktionstemperatur liegt dabei vorzugsweise zwischen 160 und 280 °C. Der Druck wird im Verlaufe der Umsetzung allmählich reduziert, der Enddruck liegt dabei vorzugsweise unter 200 mbar. Bei diesem Druck wird die Reaktion bis zum gewünschten Umsetzungsgrad weitergeführt.

Bei der einstufigen Verfahrensführung kann vorzugsweise die gesamte Umsetzung unter Verwendung von Zeolith-Katalysatoren durchgeführt werden. Es können im Verlauf der Umsetzung auch unterschiedliche Katalysatoren eingesetzt werden, diese Ausführungsform ist jedoch nicht bevorzugt. Die nach dem einstufigen Verfahren hergestellten Polyesteralkohole weisen üblicherweise die oben beschriebenen Hydroxylzahlen und Säurezahlen kleiner 2 mgKOH/g auf.

Bei der zweistufigen Herstellung der Polyesteralkohole weisen die Reaktionsprodukte der Stufe a) vorzugsweise ein zahlenmittleres Molekulargewicht im Bereich von 200 g/mol bis 10000 g/mol, besonders bevorzugt im Bereich von 500 - 5000 g/mol auf.

Die Säurezahlen der in Schritt a) hergestellten Basis-Polyesteralkohole liegen vorzugsweise im Bereich von weniger als 10 g KOH / kg, stärker bevorzugt im Bereich von weniger als 5 g KOH / kg, insbesondere im Bereich von weniger als 2 g KOH/kg. Die Säurezahl dient zur Bestimmung des Gehalts des Polyesterols an freien organischen Säuren. Die Säurezahl wird ermittelt durch die Anzahl der mg KOH (bzw. g KOH), die zur Neutralisation von 1 g (bzw. 1 kg) der Probe verbraucht wird.

Die Funktionalität der in Schritt a) hergestellten Basis-Polyesteralkohole liegt, in Abhängigkeit von den eingesetzten Rohstoffen, vorzugsweise im Bereich von mindestens 1,9 bis 4,0, stärker bevorzugt im Bereich von 2,0 bis 3,0.

Wie beschrieben, kann die Stufe a) unter Verwendung von Zeolith-Katalysatoren durchgeführt werden. Es ist jedoch auch möglich, ohne Katalysatoren oder vorzugs-weise unter Verwendung von üblichen Veresterungskatalysatoren zu arbeiten. Beispiele für übliche Veresterungskatalysatoren sind vorzugsweise metallorganische Verbindungen, wie Titantetrabutylat, Zinndioktoat oder Dibutylzinndilaurat, eine Säure, wie Schwefelsäure, p-Toluolsulfonsäure oder eine Base, wie Kaliumhydroxid oder Natrium-Methanolat eingesetzt. Diese Veresterungskatalysatoren sind in der Regel homogen und verbleiben nach Beendigung der Reaktion im Allgemeinen im Polyesteralkohol. Die Umsetzung wird hierbei bei 160 - 280 °C, vorzugsweise bei 200-260°C, durchgeführt.

Der zweite Verfahrensschritt (Schritt b)) des zweistufigen Verfahrens wird vorzugsweise ausschließlich mittels Zeolith-Katalysatoren durchgeführt. In Schritt b) erfolgt entweder die
1. mittels Zeolith-Katalysatoren katalysierte Umesterung ohne zusätzliche Glykolyse,
2. die mittels Zeolith-Katalysatoren katalysierte Glykolyse ohne die zusätzliche Umesterung oder
3. eine Mischreaktion aus mittels Zeolith-Katalysatoren katalysierter Umesterung und mittels Zeolith-Katalysatoren katalysierter Glykolyse bzw. Alkoholyse.

Bei der mittels Zeolith-Katalysatoren katalysierten Umesterung (siehe Nr. 1) werden zwei oder mehrere Basis-Polyesteralkohole aus Schritt a) mit einer ausreichenden Menge an Zeolith-Katalysatoren versetzt, wobei dann aber keine zusätzlichen mehrwertigen Polyhydroxy-Verbindungen (Diole, Glykole) zugegeben werden. In diesem Fall wird ein neues Polyesterol gebildet, welches im Idealfall ein statistisches Copolymer aus den Monomeren aller eingesetzten Basis-Polyesteralkohole ist.

Bei der mittels Zeolith-Katalysatoren katalysierten Glykolyse wird nur ein Basis-Polyesteralkohol aus Schritt a) mit einem oder mehreren Polyhydroxy-Verbindungen, vorzugsweise mit Diolen oder Polyolen, und einer geeigneten Menge Zeolith-Katalysators umgesetzt. In diesem Fall wird in der Regel das mittlere Molekulargewicht des Basis-Polyesteralkohols durch die Glykolyse bzw. durch die Alkoholyse eines Teils der Ester-Bindungen reduziert.

Alternativ kann in Verfahrensschritt b) auch eine Mischreaktion aus mittels Zeolith-Katalysatoren katalysierter Umesterung und mittels Zeolith-Katalysatoren katalysierter Glykolyse bzw. Alkoholyse stattfinden. Hierbei wird eine Abmischung aus mindestens zwei Basis-Polyesteralkoholen aus Schritt a) und mindestens einer mehrwertigen Polyhydroxy-Verbindung, vorzugsweise Diole oder Polyole, mit einer geeigneten Menge des Zeolith-Katalysators umgesetzt. Die Änderung des mittleren Molekulargewichtes oder der anderen Stoffparameter der Basis-Polyesteralkohole, wie Viskosität, Säurezahl oder Schmelzpunkt, hängt bei dieser Variante des Verfahrensschritts b) von den im Einzelfall eingesetzten Komponenten ab, insbesondere von der Art und Menge der eingesetzten Basis-Polyesteralkohole und von der Art und Menge der eingesetzten Polyhydroxy-Verbindungen.

Die Eigenschaften des Endproduktes aus Schritt b) hängen ebenfalls davon ab, ob die Umesterung bzw. Glykolyse gemäß Schritt b) vollständig erfolgt ist. Die Vollständigkeit der Umesterung bzw. Glykolyse gemäß Schritt b) hängt wiederum von der Reaktionszeit ab, wobei lange Reaktionszeiten für eine vollständige Umesterung bzw. Glykolyse sorgen. Vorzugsweise werden die Reaktionszeiten für den Umesterungsschritt b) so gewählt, dass sich letztlich Polyesteralkohole ergeben, die möglichst ähnliche Eigenschaften besitzen wie solche Polyesteralkohole, die nach dem klassischen einstufigen Hochtemperatur-Polykondensationsverfahren hergestellt wurden. Die Reaktionszeit der Umesterung bzw. Glykolyse gemäß Schritt b) kann zwischen 1 bis 36 Stunden, vorzugsweise zwischen 2 bis 24 Stunden betragen.

Die Umsetzung nach Verfahrensschritt b) kann - ähnlich wie die nach Verfahrensschritt a) - in Anwesenheit eines Lösungsmittels oder in Abwesenheit eines Lösungsmittels (Umsetzung "in Masse") durchgeführt werden.

Wird die Umsetzung nach Verfahrensschritt b) in Anwesenheit eines Lösungsmittels durchgeführt, so können alle bekannten geeigneten Lösungsmittel eingesetzt werden, insbesondere die Lösungsmittel Toluol, Dioxan, Hexan, Tetrahydrofuran, Cyclohexan, Xylol, Dimethylsulfoxid, Dimethylformamid, N-Methyl-pyrrolidon, Chloroform. Die Wahl des Lösungsmittels hängt im Einzelfall von den eingesetzten Edukten (den Basis-Polyesteralkoholen und den Polyhydroxy-Verbindungen) und insbesondere von deren Löslichkeitseigenschaften ab. Die Umsetzung nach Verfahrensschritt b) in Anwesenheit eines Lösungsmittels hat jedoch den Nachteil, dass er zusätzliche Teilverfahrensschritte umfasst, nämlich das Lösen des mindestens einen Basis-Polyesteralkohols im Lösungsmittel und das Entfernen des Lösungsmittels nach der Umsetzung. Weiterhin kann das Lösen des mindestens einen Basis-Polyesteralkohols im Lösungsmittel je nach den Hydrophobie des Basis-Polyesteralkohols problematisch sein und gegebenenfalls die Ausbeute schmälern.

In einer weiteren bevorzugten Ausführungsform des Verfahrens werden in Verfahrensschritt b) vorzugsweise solche Basis-Polyesteralkohole und gegebenenfalls zusätzliche Polyhydroxylverbindungen eingesetzt, die gemeinsam einen Wassergehalt von weniger als 0,1 Gew.-%, vorzugsweise von weniger als 0,05 Gew.-%, stärker bevorzugt von weniger als 0,03 Gew.%, insbesondere von weniger als 0,01 Gew.-% besitzen. Bei höheren Wassergehalten während des Verfahrensschritts b) findet neben der Umesterung auch Hydrolyse statt, so dass die Säurezahl des Polyesteralkohols während des Schritts b) in unerwünschter Weise ansteigen würde. Die Durchführung des Schritts b) des erfindungsgemäßen Verfahrens bei einem Wassergehalt von weniger als 0,1 Gew.-%, vorzugsweise von weniger als 0,05 Gew.-%, stärker bevorzugt von weniger als 0,03 Gew.-%, insbesondere von weniger als 0,01 Gew.-% führt somit zur Herstellung von Spezial-Polyesteralkoholen mit niedriger Säurezahl als Endprodukte. Polyesteralkohole mit niedriger Säurezahl sind im allgemeinen hydrolysestabiler als Polyesteralkohole mit hoher Säurezahl, da freie Säuregruppen die Rückreaktion, d.h. die Hydrolyse, katalysieren.

Eine Herstellung von Polyesteralkoholen mit Wassergehalten von mehr als 0,1 Gew.-% führt zu Polyesteralkoholen mit einer Säurezahl von größer als 10 mg KOH/g. Polyesteralkohole mit so hohen Säurezahlen (größer als 10 mg KOH/g) sind jedoch für die meisten industriellen Anwendungen, insbesondere zur Verwendung bei der Herstellung von Polyesteralkoholen, nicht oder nur schlecht geeignet.

Auch Polyesteralkohole nehmen je nach Luftfeuchtigkeitsgehalt und Temperatur mindestens 0,01 Gew.-%, in der Regel aber mindestens 0,02 Gew.-%, in einigen Fällen auch mehr als 0,05 Gew.-% Wasser auf. Je nach Umsetzungsgrad und Molekulargewicht der eingesetzten Basis-Polyesteralkohole ist diese Wasserkonzentration höher als die Gleichgewichtswasserkonzentration. Wird das Polyesteralkohole vor dem Verfahrensschritt b) nicht getrocknet, so setzt unweigerlich die Hydrolyse des Polyesteralkohols ein.

Der Wassergehalt der Basis-Polyesteralkohole, die in Schritt b) eingesetzt werden, werden daher vorzugsweise vor der Umesterung nach Verfahrensschritt b) getrocknet. Auch die gegebenenfalls einzusetzende mehrwertige Polyhydroxylverbindung, beispielsweise das Diol, wird vorzugsweise vor der Umesterungsreaktion getrocknet, um den oben genannten geringen Wassergehalt bei der Umesterung zu erreichen. Die Trocknung kann anhand gängiger Trocknungsverfahren nach dem Stand der Technik durchgeführt werden, so beispielsweise durch Trocknung über Molekularsieb oder Fallfilmverdampfer. Alternativ können Basis-Polyesteralkohole mit niedrigen Wassergehalten, vorzugsweise von weniger als 0,1 Gew.-%, bevorzugter von weniger als 0,05 Gew.-%, bevorzugter von weniger als 0,03 Gew.-%, insbesondere von weniger als 0,01 Gew.-%, auch dadurch erhalten werden, dass die Umsetzung gemäß Verfahrensschritt a) sowie auch ein eventueller Schritt der Zwischenlagerung des mindestens einen Basis-Polyesteralkohols vollständig unter inerten Bedingungen, so beispielsweise in einer Inertgas-Atmosphäre, vorzugsweise in einer Stickstoff-Atmosphäre, durchgeführt wird. In diesem Falle haben die Basis-Polyesteralkohole von Anfang an keine Möglichkeit, größere Mengen Wasser aus der Umgebung aufzunehmen. Ein gesonderter Trocknungsschritt könnte sich dann erübrigen.

In einer weiteren bevorzugten Ausführungsform des Verfahrens wird daher das mindestens eine Basis-Polyesteralkohol aus Verfahrensschritt a) vor der Umsetzung nach Verfahrensschritt b) zwischengelagert, vorzugsweise in einer Inertgas-Atmosphäre, um den Wassergehalt gering zu halten. Aus den zwischengelagerten Basis-Polyesteralkoholen kann dann eine Mischung zweier oder mehrerer Basis-Polyesteralkohole in geeigneten Mengenverhältnissen zusammengestellt werden, um nach der Umesterung und nach der eventuellen zusätzlichen Glykolyse mit Polyhydroxy-Verbindungen einen bestimmten Spezial-Polyesteralkohol mit ganz spezifischen physikalischen Eigenschaften und mit spezifischer Struktur zu erhalten.

Die nach dem zweistufigen erfindungsgemäßen Verfahren hergestellten Polyesteralkohole haben in der Regel relativ niedrige Säurezahlen, nämlich vorzugsweise Säurezahlen von weniger als 3 mg KOH pro Gramm Polyesterol, stärker bevorzugt von weniger als 2 mg KOH pro Gramm Polyesteralkohol, insbesondere von weniger als 1 mg KOH pro Gramm Polyesteralkohol.

Diese niedrigen Säurezahlen werden insbesondere dadurch gewährleistet, dass Verfahrensschritt b) vorzugsweise bei einem Wassergehalt von weniger als 0,1 Gew.-%, bevorzugter von weniger als 0,05 Gew.-%, bevorzugter von weniger als 0,03 Gew.-%, insbesondere von weniger als 0,01 Gew.-% durchgeführt wird.

Wie beschrieben, können die Polyesteralkohole auch durch ringöffnende Polymerisation von cyclischen Estern, vorzugsweise von Lactonen, insbesondere ε-Caprolacton, hergestellt werden. Diese cyclischen Ester können allein oder im Gemisch mit den oben beschriebenen Ausgangsstoffen eingesetzt werden.

Zur Durchführung des Verfahrensschrittes a) können sämtliche Reaktoren, deren Einsatz für klassische Hochtemperatur-Polykondensationen bekannt ist, eingesetzt werden (siehe hierzu Ullmann Encyclopedia (Electronic Release), Kapitel: Polyesters, Absatz: Polyesters as Intermediates for Polyurethane).

Die Durchführung des Verfahrensschrittes b) erfolgt zumeist in einem Temperaturbereich von 50-160°C, vorzugsweise unter Normaldruck. Bevorzugt wird die Reaktion in einer inerten Atmosphäre unter Ausschluss von Luftfeuchtigkeit, beispielsweise durch Überleitung von Stickstoff ausgeführt. Die Durchführung von Verfahrensschritt b) erfolgt vorzugsweise in einem beheizten Rührkessel oder Festbettreaktor. Das erfindungsgemäße Verfahren kann diskontinuierlich, halbkontinuierlich oder kontinuierlich durchgeführt werden.

Die nach dem erfindungsgemäßen Verfahren hergestellten Polyesteralkohole können vorzugsweise durch Umsetzung mit Isocyanaten zu Polyurethanen, wie Polyurethan-Hartschaumstoffen, Polyurethan-Weichschaumstoffen, Integralschaumstoffen, wie beispielsweise Schuhsohlen, verarbeitet werden. Ein besonders bevorzugtes Einsatzgebiet ist die Herstellung von thermoplastischen Polyurethan-Elastomeren, auch als TPU bezeichnet.

Verfahren zur Herstellung der Polyurethane sind ebenfalls allgemein bekannt. Beispielsweise können thermoplastische Polyurethane durch Umsetzung von Diisocyanaten mit Verbindungen mit mindestens zwei mit Isocyanatgruppen reaktiven Wasserstoffatomen, vorzugsweise difunktionellen Alkoholen und gegebenenfalls Kettenverlängerungsmitteln mit einem Molekulargewicht von 50 bis 499 gegebenenfalls in Gegenwart von Katalysatoren und/oder üblichen Hilfsstoffen herstellt werden.

Als Diisocyanate werden übliche aromatische, aliphatische, cycloaliphatische und/oder araliphatische Isocyanate, bevorzugt Diisocyanate eingesetzt werden, beispielsweise 2,2'-, 2,4'- und/oder 4,4'-Diphenylmethandiisocyanat (MDI), 1,5-Naphthylendiisocyanat (NDI), 2,4- und/oder 2,6-Toluylendiisocyanat (TDI), Diphenylmethandiisocyanat3,3'-Dimethyl-diphenyl-diisocyanat, 1,2-Diphenylethandiisocyanat und/oder Phenylendiisocyanat, Tri-, Tetra-, Penta-, Hexa-, Hepta- und/oder Oktamethylendiisocyanat, 2-Methyl-pentamethylen-diisocyanat-1,5, 2-Ethyl-butylen-diisocyanat-1,4, Pentamethylen-diisocyanat-1,5, Butylen-diisocyanat-1,4, 1-Isocyanato-3,3,5-trimethyl-5-iso-cyanatomethyl-cyclohexan (Isophoron-diisocyanat, IPDI), 1-Methyl-2,4- und/oder - 2,6-cyclohexan-diisocyanat, 4,4'-, 2,4'- und/oder 2,2'-Dicyclohexylmethan-diisocyanat (H12MDI) 2,6-Diisocyanatohexancarbonsäureester, 1,4- und/oder 1,3-Bis(iso-cyanatomethyl)cyclohexan (HXDI), 1,4-Cyclohexan-diisocyanat, 1-Methyl-2,4-und/oder -2,6-cyclohexan-diisocyanat und/oder 4,4'-, 2,4'- und 2,2'-Dicyclohexyl-methan-diisocyanat, bevorzugt 2,2'-, 2,4'- und/oder 4,4'-Diphenylmethandiisocyanat (MDI), 1,5-Naphthylendiisocyanat (NDI), 2,4- und/oder 2,6-Toluylendiisocyanat (TDI), Hexamethylendiisocyanat, 4,4'-, 2,4'- und/oder 2,2'-Dicyclohexylmethan-diisocyanat (H12MDI), und/oder IPDI, insbesondere 4,4'- MDI und/oder Hexamethylendiisocyanat und/oder H12MDI.

Als gegenüber Isocyanaten reaktive Verbindungen werden, wie beschrieben, die erfindungsgemäßen Polyesteralkohole eingesetzt. In Gemisch mit diesen können allgemein bekannte, gegenüber Isocyanaten reaktive Verbindungen eingesetzt werden, beispielsweise Polyesterole, Polyetherole und/oder Polycarbonatdiole, die üblicherweise auch unter dem Begriff "Polyole" zusammengefaßt werden, mit Molekulargewichten von 500 bis 12000 g/mol, bevorzugt 600 bis 6000, insbesondere 800 bis 4000, und bevorzugt einer mittleren Funktionalität von 1,8 bis 2,3, bevorzugt 1,9 bis 2,2, insbesondere 2. Bevorzugt setzt man ausschließlich die erfindungsgemäßen Polyesteralkoholen als gegenüber Isocyanaten reaktive Verbindungen ein.

Zu den gegenüber Isocyanaten reaktiven Verbindungen gehören auch die Kettenverlängerungsmittel. Als Kettenverlängerungsmittel können allgemein bekannte aliphatische, araliphatische, aromatische und/oder cycloaliphatische Verbindungen mit einem Molekulargewicht von 50 bis 499, bevorzugt 2-funktionelle Verbindungen, eingesetzt werden, beispielsweise Alkandiole mit 2 bis 10 C-Atomen im Alkylenrest, bevorzugt Butandiol-1,4, Hexandiol-1,6, Propanediol-1,3, Ethylenglykole-1,2 und/oder Di-, Tri-, Tetra-, Penta-, Hexa-, Hepta-, Okta-, Nona- und/oder Dekaalkylenglykole mit 3 bis 8 Kohlenstoffatomen, bevorzugt unverzweigte Alkandiole, insbesondere Propan-1,3-diol, Butan-1,4-diol und Hexanediol-1,6.

Üblicherweise werden Katalysatoren eingesetzt, welche die Reaktion zwischen den NCO-Gruppen der Diisocyanate und den Hydroxylgruppen der Aufbaukomponenten beschleunigen, sind die nach dem Stand der Technik bekannten und üblichen tertiären Amine, wie z.B. Triethylamin, Dimethylcyclohexylamin, N-Methylmorpholin, N,N'-Dimethylpiperazin, 2-(Dimethylaminoethoxy)-ethanol, Diazabicyclo-(2,2,2)-octan und ähnliche sowie insbesondere organische Metallverbindungen wie Titansäureester, Eisenverbindungen wie z.B. Eisen-(III)- acetylacetonat, Zinnverbindungen, z.B. Zinndiacetat, Zinndioctoat, Zinndilaurat oder die Zinndialkylsalze aliphatischer Carbonsäuren wie Dibutylzinndiacetat, Dibutylzinndilaurat oder ähnliche. Die Katalysatoren werden üblicherweise in Mengen von 0,00001 bis 0,1 Gew.-Teilen pro 100 Gew.-Teile Polyhydroxylverbindung eingesetzt.

Neben Katalysatoren können den Aufbaukomponenten bis auch übliche Hilfsstoffe hinzugefügt werden. Genannt seien beispielsweise oberflächenaktive Substanzen, Flammschutzmittel, Keimbildungsmittel, Gleit- und Entformungshilfen, Farbstoffe und Pigmente, Inhibitoren, Stabilisatoren gegen Hydrolyse, Licht, Hitze, Oxidation oder Verfärbung, Schutzmittel gegen mikrobiellen Abbau, anorganische und/oder organische Füllstoffe, Verstärkungsmittel und Weichmacher.

Nähere Angaben über die oben genannten Hilfsmittel- und Zusatzstoffe sind der Fachliteratur zu entnehmen, z.B. aus Plastics Additive Handbook, 5th edition, H. Zweifel, ed, Hanser Publishers, München, 2001. Alle in dieser Schrift genannten Molekulargewichte weisen die Einheit [g/mol] auf.

Zur Einstellung von Härte der TPU können die Aufbaukomponente Polyole- und Kettenverlängerer in relativ breiten molaren Verhältnissen variiert werden. Bewährt haben sich molare Verhältnisse von Polyolen zu insgesamt einzusetzenden Kettenverlängerungsmitteln von 10/1 bis 1/10, insbesondere 1/1 bis 1/4, wobei die Härte der TPU mit zunehmendem Gehalt an Kettenverlängerungsmittel ansteigt.

Die Herstellung der Polyurethane kann nach den bekannten Verfahren diskontinuierlich oder kontinuierlich, beispielsweise mit Reaktionsextrudern oder dem Bandverfahren nach one-shot oder dem Prepolymerverfahren, bevorzugt nach dem one-shot-Verfahren erfolgen. Beim Prepolymerverfahren können die zur Reaktion kommenden Komponenten Isocyanate, Polyole und gegebenenfalls Kettenverlängerungsmittel, Katalysatoren und/oder Hilfsmittel nacheinander oder gleichzeitig miteinander vermischt werden, wobei die Reaktion unmittelbar einsetzt. Beim Extruderverfahren werden die Aufbaukomponenten Isocyanate, Polyole und gegebenenfalls Kettenverlängerungsmittel, Katalysatoren und/oder Hilfsmittel einzeln oder als Gemisch in den Extruder eingeführt, üblicherweise bei Temperaturen von 100°C bis 280°C, vorzugsweise 140°C bis 250°C zur Reaktion gebracht. Das erhaltene TPU wird extrudiert, abgekühlt und granuliert.

Durch das erfindungsgemäße Verfahren war es überraschenderweise möglich, die zur Herstellung der Polyesteralkohole nötige Reaktionszeit zu senken. Die Polyesteralkohole zeichnen sich durch eine verbesserte Lagerstabilität und eine niedrige Farbzahl aus. Es gab keinerlei Nachteile in den Verarbeitungseigenschaften und in den Kennwerten der unter Verwendung der Polyesteralkohole hergestellten Polyurethane.

Die Erfindung soll an den nachfolgenden Beispielen näher erläutert werden.

### Beispiel 1 Herstellung eines strangförmigen Zeoliths

3,0 kg Pulver eines Titanzeoliths wurden mit 2,5 kg Ludox^{®} AS 40, 3,83 kg einer 33,5%igen Polystyroldispersion, 120 g Walocel^{®}, 40 g Polyethylenoxid und 1000 g Wasser für 65 min in einem Koller vermischt. Anschließend wurde die Mischung bei einem Druck von 140 bar zu 1,5 mm Stränglingen extrudiert. Die Stränglinge wurden für 16 h bei 120 °C getrocknet und abschließend für 5 h bei 490 °C an Luft calciniert. Es wurden 3,75 kg Stränglinge erhalten mit einem Ti-Gehalt von 1,5 % und einem Si-Gehalt von 44,0 %.

### Herstellung der Polyesteralkohole

### Vergleichsbeispiel 1

6040,1 g Adipinsäure, 1406,8 g Ethylenglykol, 2042,6 g Butandiol-1,4, 1ppm Titantetrabutanolat und 5ppm Zinn octoat wurden in einen Rundkolben mit einem Volumen von 12 Litern gefüllt. Die Mischung wurde unter Rühren auf 180 °C erhitzt und 3 Stunden bei dieser Temperatur belassen. Dabei wurde das entstehende Wasser durch Destillation entfernt.

Danach wurde die Mischung auf 240 °C erwärmt und bei dieser Temperatur belassen unten ein Vakuum auf 40 mbar, bis eine Säurezahl kleiner 1 mgKOH/g erreicht war.

Der entstandene farblose, flüssige Polyesteralkohol hatte folgende Kennwerte:

| | |
|---|---|
| Hydroxylzahl: | 56,5 mgKOH/g |
| Säurezahl: | 0,10 mgKOH/g |
| Viskosität: | 670 mPa.s bei 75 °C |
| Wassergehalt: | 0,04% |
| Farbzahl: | 64 APHA/Hazen |
| Zykluszeit: | 14 Stunden |
| Metallgehalt im Polyesteralkohol: | Ti: 0,21 ppm; Sn: 1,2 ppm |

### Vergleichsbeispiel 2

5301,6 g Adipinsäure, 1586,4 g Hexanediol-1,6, 2419,5 g Butandiol-1,4 und 10 ppm Zinnoctoat wurden in einen Rundkolben mit einem Volumen von 12 Litern gefüllt. Die Mischung wurde unter Rühren auf 180 °C erhitzt und 3 Stunden bei dieser Temperatur belassen. Dabei wurde das entstehende Wasser durch Destillation entfernt.

Danach wurde die Mischung auf 240 °C erwärmt und bei dieser Temperatur belassen unten ein Vakuum auf 40 mbar, bis eine Säurezahl kleiner 1 mgKOH/g erreicht war.

Der entstandene farblose, flüssige Polyesteralkohol hatte folgende Kennwerte:

| | |
|---|---|
| Hydroxylzahl: | 56 mgKOH/g |
| Säurezahl: | 0,27 mgKOH/g |
| Viskosität: | 690 mPa.s bei 75 °C |
| Wassergehalt: | 0,1% |
| Farbzahl: | 50 APHA/Hazen |
| Zykluszeit: | 11 Stunden |
| Metallgehalt im Polyesteralkohol: | Sn: 2,6 ppm |

### Beispiel 1

6040,1 g Adipinsäure, 1406,8 g Ethylenglykol, 2042,6 g Butandiol-1,4 und 18,9 g Titan-Zeolithkatalysator wurden in einen Rundkolben mit einem Volumen von 12 Litern gefüllt. Die Mischung wurde unter Rühren auf 180 °C erhitzt und 3 Stunden bei dieser Temperatur belassen. Dabei wurde das entstehende Wasser durch Destillation entfernt.

Danach wurde die Mischung auf 240 °C erwärmt und bei dieser Temperatur belassen unten ein Vakuum auf 40 mbar, bis eine Säurezahl kleiner 1 mgKOH/g erreicht war.

Nach Filtration des Titan-Zeolithkatalysators wurde ein farbloser, flüssiger Polyesteralkohol mit folgenden Kennwerten erhalten:

| | |
|---|---|
| Hydroxylzahl: | 57 mgKOH/g |
| Säurezahl: | 0,1 mgKOH/g |
| Viskosität: | 660 mPa.s bei 75 °C |
| Wassergehalt: | 0,05% |
| Farbzahl: | 12 APHA/Hazen |
| Zykluszeit: | 10 Stunden |
| Metallgehalt im Polyesteralkohol: | Ti: < 0, 1 ppm |

### Beispiel 2

5301,6 g Adipinsäure, 1586,4 g Hexanediol-1,6, 2419,5 g Butandiol-1,4 und 18,6 g Titan-Zeolithenkatalysators wurden in einen Rundkolben mit einem Volumen von 12 Litern gefüllt. Die Mischung wurde unter Rühren auf 180 °C erhitzt und 3 Stunden bei dieser Temperatur belassen. Dabei wurde das entstehende Wasser durch Destillation entfernt.

Danach wurde die Mischung auf 240 °C erwärmt und bei dieser Temperatur belassen unten ein Vakuum auf 40 mbar, bis eine Säurezahl kleiner 1 mgKOH/g erreicht war.

Nach Filtration des Titan-Zeolithkatalysators wurde ein farbloser, flüssiger Polyesteralkohol mit folgenden Kennwerten erhalten:

| | |
|---|---|
| Hydroxylzahl: | 56 mgKOH/g |
| Säurezahl: | 0,51 mgKOH/g |
| Viskosität: | 680 mPa.s bei 75 °C |
| Wassergehalt: | 0,04% |
| Farbzahl: | 18 APHA/Hazen |
| Zykluszeit: | 9 Stunden |
| Metallgehalt im Polyesteralkohol: | Ti: < 0, 1ppm |

| Polyesterol | Diol | OH Zahl (mg KOH/g) | SZ Zahl (mg KOH/g) | Viscosltät (75°C) (mPa.s) | Farbzahl (APHA/Hazen) | Zykluszeit Stunde |
|---|---|---|---|---|---|---|
| Vergleichsbeispiel 1 | Ethylene glycol, Butanediol 1,4 | 56.5 | 0,1 | 670 | 64 | 14 |
| Vergleichsbeispiel 2 | Butanediol 1,4, Hexanediol 1,6 | 56 | 0,27 | 690 | 50 | 11 |
| Beispiel 1 | Ethylene glycol, Butanediol 1,4 | 57 | 0.1 | 660 | 12 | 10 |
| Beispiel 2 | Butanediol 1.4, Hexanediol 1,6 | 56 | 0.51 | 680 | 18 | 9 |

Tabelle 1 zeigt, dass die nach dem erfindungsgemäßen Verfahren hergestellten Polyesterole in einer kürzeren Zykluszeit hergestellt werden konnten und dass die nach dem erfindungsgemäßen Verfahren hergestellten Polyesterole eine geringere Verfärbung aufweisen.

## Patentansprüche

1. Verfahren zur Herstellung von Polyesteralkoholen durch katalytische Umsetzung von mindestens einer mindestens mehrfunktionellen Carbonsäure mit mindestens einem mehrfunktionellen Alkohol und/oder durch katalytische Ring-Öffnung Polymerisation von cyclischen Estern in Gegenwart von Katalysatoren, **dadurch gekennzeichnet, dass** als Katalysator ein Zeolith eingesetzt wird, wobei das Zeolith ein Titanzeolith ist.

2. Verfahren nach Anspruch 1, **dadurch gekennzeichnet, dass** das Zeolith eine röntgenographische Zuordnung zur MFI, MOR, BEA, MWW, RRO, LEV, FER, MEL Struktur oder MFI/MEL-Mischstruktur nach "Atlas of Zeolite Framework Types", Ch. Baerlocher, W.M. Meier, D.H. Olson, 5th ed. Elsevier, 2001, aufweist.

3. Verfahren nach Anspruch 1 bis 2, **dadurch gekennzeichnet, dass** das Zeolith eineröntgenographische Zuordnung zur MFI-, MEL- oder MFI/MEL-Mischstruktur nach "Atlas of Zeolite Framework Types", Ch. Baerlocher, W.M. Meier, D.H. Olson, 5th ed. Elsevier, 2001, aufweist.

4. Verfahren nach Anspruch 1, **dadurch gekennzeichnet, dass** die gesamte Umsetzung in Gegenwart eines Titanzeoliths durchgeführt wird.

5. Verfahren nach Anspruch 1, **dadurch gekennzeichnet, dass** das Titanzeolith als Festbett eingesetzt wird.

6. Verfahren nach Anspruch 1, **dadurch gekennzeichnet, dass** das Titanzeolith als Pulver eingesetzt wird.

7. Verfahren nach Anspruch 1, **dadurch gekennzeichnet, dass** die Umsetzung zweistufig durchgeführt wird und folgende Verfahrensschritte umfasst:
a) Herstellung mindestens eines Basis-Polyesteralkohols durch die Umsetzung von jeweils mindestens einer Dicarbonsäure mit jeweils mindestens einer Polyhydroxylverbindung
b) Umsetzung des Produkts aus Schritt a) oder einer Mischung aus dem Produkt aus Schritt a), gegebenenfalls im Gemisch mit weiteren Polyhydroxylverbindungen, mit einem Titanzeolith.

8. Verfahren nach Anspruch 7, **dadurch gekennzeichnet, dass** Schritt a) in Anwesenheit eines Veresterungskatalysators durchgeführt wird.

9. Verfahren nach Anspruch 7, **dadurch gekennzeichnet, dass** Schritt a) in Anwesenheit eines Veresterungskatalysators, ausgewählt aus der Gruppe, enthaltend Toluolsulfonsäuren und metallorganische Verbindungen durchgeführt wird.

10. Verfahren nach Anspruch 7, **dadurch gekennzeichnet, dass** die metallorganischen Verbindungen solche auf Basis von Titan oder Zinn sind.

11. Verfahren nach Anspruch 7, **dadurch gekennzeichnet, dass** die metallorganischen Verbindungen Titantetrabutylat oder Zinn-(II)-octoat, Dibutylzinnlaurat und/oder Zinnchlorid sind

12. Verfahren nach Anspruch 7, **dadurch gekennzeichnet, dass** der Schritt b) kontinuierlich durchgeführt wird.

## Claims

1. A process for preparing polyester alcohols by catalytic reaction of at least one at least polyfunctional carboxylic acid with at least one polyfunctional alcohol and/or by catalytic ring-opening polymerization of cyclic esters in the presence of catalysts, wherein a zeolite is used as catalyst and the zeolite is a titanium zeolite.

2. The process according to claim 1, wherein the zeolite can be assigned X-ray-crystallographically to the MFI, MOR, BEA, MWW, RRO, LEV, FER, MEL structure or MFI/MEL mixed structure as per "Atlas of Zeolite Framework Types", Ch. Baerlocher, W.M. Meier, D.H. Olson, 5th ed. Elsevier, 2001.

3. The process according to either of claims 1 and 2, wherein the zeolite can be assigned X-ray-crystallographically to the MFI structure, MEL structure or MFI/MEL mixed structure as per "Atlas of Zeolite Framework Types", Ch. Baerlocher, W.M. Meier, D.H. Olson, 5th ed. Elsevier, 2001.

4. The process according to claim 1, wherein the entire reaction is carried out in the presence of a titanium zeolite.

5. The process according to claim 1, wherein the titanium zeolite is used as fixed bed.

6. The process according to claim 1, wherein the titanium zeolite is used as powder.

7. The process according to claim 1, wherein the reaction is carried out in two stages and comprises the following process steps:
a) preparation of at least one base polyester alcohol by reaction of in each case at least one dicarboxylic acid with in each case at least one polyhydroxyl compound,
b)reaction of the product from step a) or a mixture of the product from step a), optionally in admixture with further polyhydroxyl compounds, with a titanium zeolite.

8. The process according to claim 7, wherein step a) is carried out in the presence of an esterification catalyst.

9. The process according to claim 7, wherein step a) is carried out in the presence of an esterification catalyst selected from the group consisting of toluenesulfonic acids and metal-organic compounds.

10. The process according to claim 7, wherein the metal-organic compounds are based on titanium or tin.

11. The process according to claim 7, wherein the metal-organic compounds are titanium tetrabutoxide or tin(II) octoate, dibutyltin laurate and/or tin chloride.

12. The process according to claim 7, wherein step b) is carried out continuously.

## Revendications

1. Procédé de fabrication de polyester-alcools par réaction catalytique d'au moins un acide carboxylique au moins polyfonctionnel avec au moins un alcool polyfonctionnel et/ou par polymérisation catalytique par ouverture de cycle d'esters cycliques en présence de catalyseurs, **caractérisé en ce qu'**une zéolithe est utilisée en tant que catalyseur, la zéolithe étant une zéolithe au titane.

2. Procédé selon la revendication 1, **caractérisé en ce que** la zéolithe présente une classification radiographique selon la structure MFI, MOR, BEA, MWW, RRO, LEV, FER, MEL ou la structure mixte MFI/MEL selon « Atlas of Zeolite Framework Types », Ch. Baerlocher, W. M. Meier, D. H. Olson, 5th ed. Elsevier, 2001.

3. Procédé selon les revendications 1 à 2, **caractérisé en ce que** la zéolithe présente une classification radiographique selon la structure MFI, MEL ou la structure mixte MFI/MEL selon « Atlas of Zeolite Framework Types », Ch. Baerlocher, W. M. Meier, D. H. Olson, 5th ed. Elsevier, 2001.

4. Procédé selon la revendication 1, **caractérisé en ce que** l'ensemble de la réaction est réalisé en présence d'une zéolithe au titane.

5. Procédé selon la revendication 1, **caractérisé en ce que** la zéolithe au titane est utilisée sous la forme d'un lit fixe.

6. Procédé selon la revendication 1, **caractérisé en ce que** la zéolithe au titane est utilisée sous la forme d'une poudre.

7. Procédé selon la revendication 1, **caractérisé en ce que** la réaction est réalisée en deux étapes et comprend les étapes de procédé suivantes :
a) la fabrication d'au moins un polyester-alcool de base par la réaction à chaque fois d'au moins un acide dicarboxylique avec à chaque fois au moins un composé polyhydroxyle,
b) la réaction du produit de l'étape a) ou d'un mélange du produit de l'étape a), éventuellement en mélange avec d'autres composés polyhydroxyle, avec une zéolithe au titane.

8. Procédé selon la revendication 7, **caractérisé en ce que** l'étape a) est réalisée en présence d'un catalyseur d'estérification.

9. Procédé selon la revendication 7, **caractérisé en ce que** l'étape a) est réalisée en présence d'un catalyseur d'estérification choisi dans le groupe contenant les acides toluènesulfoniques et les composés métallo-organiques.

10. Procédé selon la revendication 7, **caractérisé en ce que** les composés métallo-organiques sont à base de titane ou d'étain.

11. Procédé selon la revendication 7, **caractérisé en ce que** les composés métallo-organiques sont le tétrabutylate de titane ou l'octoate d'étain (II), le laurate de dibutylétain et/ou le chlorure d'étain.

12. Procédé selon la revendication 7, **caractérisé en ce que** l'étape b) est réalisée en continu.
